# EUROPEAN PATENT SPECIFICATION

(11) **EP 0 670 736 B1**
(45) Date of publication and mention of the grant of the patent: **19.09.2001**
(21) Application number: 93923789.7
(22) Date of filing: 04.10.1993
(51) Int. Cl.: A61K 51/08

(54) **LABELLED INTERLEUKIN-8 AND MEDICAL USES THEREOF**
Markiertes Interleukin-8 und seine medizinische Anwendung
INTERLEUKINE-8 MARQUEE ET SES UTILISATIONS MEDICALES

(30) Priority: 05.10.1992 US 956863
(43) Date of publication of application: 13.09.1995
(73) Proprietor: MALLINCKRODT MEDICAL, INC., St. Louis, MO 63042 (US); The Regents of The University of Michigan Technology Management Wolverine Tower Office, Ann Arbor, Michigan 48109-1280 (US)
(72) Inventor: LYLE, Leon, R., Webster Groves, MO 63119 (US); KUNKEL, Steven, L., Ann Arbor, MI 48104 (US)
(74) Representative: Kinzebach, Werner, Dr.
(86) International application number: PCT/US93/09554
(87) International publication number: WO 94/07535

(56) References cited:
- EP-A- 0 398 143
- WO-A-92/04372
- JOURNAL OF BIOLOGICAL CHEMISTRY. (MICROFILMS), vol.265, no.1, 5 January 1990, BALTIMORE, MD US pages 183 - 189 AJOY K. SAMANTA ET AL. 'INTERLEUKIN 8 (MONOCYTE-DERIVED NEUTROPHIL CHEMOTACTIC FACTOR) DYNAMICALLY REGULATES ITS OWN RECEPTOR EXPRESSION ON HUMAN NEUTROPHILS.'
- BIOCHEMICAL AND BIOPHYSICAL RESEARCH COMMUNICATIONS, vol.180, no.1, 15 October 1991, DULUTH, MINNESOTA US pages 301 - 307 W. SCHNITZEL ET AL. 'NEUTROPHIL ACTIVATING PEPTIDE-2 BINDS WITH TWO AFFINITIES TO RECEPTOR(S) ON HUMAN NEUTROPHILS'
- CHEMICAL ABSTRACTS, vol. 119, no. 21, 22 November 1993, Columbus, Ohio, US; abstract no. 223971u, & CYTOKINE, vol.4, no.5, 1992, PHILADELPHIA pages 347 - 352 ANTAL ROT 'BINDING OF NEUTROPHIL ATTRACTANT/ACTIVATION PROTEIN-1 (INTERLEUKIN 8) TO RESIDENT DERMAL CELLS.'
- CHEMICAL ABSTRACTS, vol. 120, no. 9 Columbus, Ohio, US; abstract no. 104626w, & EUR. CYTOKINE NETWORK, vol.4, no.3, 1993 pages 197 - 204 N. VITA ET AL. 'FUNCTIONAL LINKAGE OF THE GRO.BETA AND IL-8 RECEPTORS ON THE SURFACE OF HUMAN NEUTROPHILS'
- JOURNAL OF BIOLOGICAL CHEMISTRY. (MICROFILMS), vol.268, no.1, 1993, BALTIMORE, MD US pages 541 - 546 R. HORUK ET AL. 'PURIFICATION, RECEPTOR BINDING ANALYSIS, AND BIOLOGICAL CHARACTERIZATION OF HUMAN MELANOMA GROWTH STIMULATING ACTIVITY (MGSA)'
- JOURNAL OF BIOLOGICAL CHEMISTRY. (MICROFILMS), vol.268, no.17, 15 June 1993, BALTIMORE, MD US pages 12247 - 12249 KULDEEP NEOTE ET AL. 'IDENTIFICATION OF A PROMISCUOUS INFLAMMATORY PEPTIDE RECEPTOR ON THE SURFACE OF RED BLOOD CELLS.'

## Description

### Field of the Invention

The present invention relates to the field of labelled peptides, peptides carrying radioactive agents, and medical uses thereof. In particular, the present invention relates to the use of labelled CXC chemokines and labelled peptides having specificity for a receptor of Interleukin-8 for preparing pharmaceutical compositions; as well as to compositions comprising certain forms of CXC chemokine.

### Description of the Background Art

Labelled peptides and peptides carrying radioactive agents have various therapeutic and diagnostic medical uses. Peptides carrying radioactive agents are known to be therapeutically useful in the treatment of tumors.

An important diagnostic use of labelled peptides is as imaging agents. For example, U.S. Patent No. 4,925,869 to Rubin et al. discloses detection of an inflammation site in an individual by administering to the individual a labelled immunoglobulin or fragment thereof. The labelled immunoglobulin accumulates at the site of inflammation, thereby permitting radiographic imaging of the site utilizing known imaging techniques.

Other publications which describe the imaging of sites of infection or inflammation, utilizing labelled peptides and peptides carrying radioactive agents, include International Patent Publication Nos. WO 90/10463 and WO 90/13317.

There remains a need in the art for labelled peptides and peptides carrying radioactive agents which can be utilized for medical purposes.

### SUMMARY OF THE INVENTION

In accordance with the present invention, a labelled CXC chemokine is utilized to prepare a pharmaceutical composition to image a target site in an animal's body. The labelled CXC chemokine is introduced into the animal's body, and allowed to accumulate at a target site which has receptor molecules complementary to Interleukin-8. The accumulated, labelled CXC chemokine then is detected so as to image the target site.

According to a first embodiment the present invention relates to the use of a labelled CXC chemokine or labelled peptide having specificity for a receptor of Interleukin-8 for preparing a pharmaceutical composition for imaging a target site in an animal body, said target site including Interleukin-8 receptor molecules. Preferably, said target site is an inflamed or diseased portion of said animal's body. Said labelled CXC chemokine is a preferably radiolabelled CXC chemokine, as for example chemokine labelled with indium-111, iodine-123, technetium-99m or copper-62. Said indium-111 is preferably carried by a lysine of said CXC chemokine; or said CXC chemokine is labelled with indium-111 utilizing a chelating agent, as for example C-DTPA, and the CXC chemokine may be human Interleukin-8.

The above-mentioned iodine-123 is preferably carried by a tyrosine, iodophenyl derivative or lysine of said CXC chemokine. In another preferred embodiment said CXC chemokine, as for example human Interleukin-8, is labelled with iodine-123 utilizing choramine-T hydrate.

The CXC chemokine, as for example Interleukin-8, may also be labelled with technetium-99m. It is preferred that said technetium-99m is carried by a lysine or an added free cysteine of said CXC chemokine.

In another preferred embodiment said CXC chemokine is labelled utilizing a chelating agent, said chelating agent preferably being selected from a triamide thiolate ligand, a diamide dithiolate ligand or a diamide diphenolic ligand; and particularly preferred CXC chemokine is labelled with technetium-99m or copper-62 and said chelating agent is a triamide thiolate ligand.

Accoding to another embodiment the invention relates to the use of a CXC chemokine or peptide carrying a labelling agent, as for example a radioactive agent, which peptide recognizes Interleukin-8 receptor molecules of said CXC chemokine, for preparing a pharmaceutical composition for delivering a labelling agent to a target site of an animal's body by introducing into an animal's body said CXC chemokine or peptide carrying a labelling agent, and allowing the labelling agent-carrying CXC chemokine or peptide to accumulate at a target site which includes Interleukin-8 receptor molecules so as to deliver said labelling agent to said target site.

Another preferred CXC chemokine is Neutrophil-activating Peptide-2 (NAP-2).

According to a further embodiment the present invention provides a composition comprising a CXC chemokine carrying a labelling agent selected from the group consisting of indium-111, iodine-123 and technetium-99m; as well as a composition comprising CXC chemokine conjugated with a chelating agent wherein said chelating agent preferably is a triamide thiolate ligand, diamide dithiolate ligand or diamide diphenolic ligand.

According to a preferred use of the invention, said chemokine is a CXC chemokine including an ELR amino acid sequence, wherein E stands for glutamic acid, L stands for leucine and R stands for arginine, and wherein said ELR amino acid sequence immediately precedes a CXC sequence in said chemokine, wherein C stands for cysteine and X stands for an amino acid other than cysteine, said CXC sequence comprising four cysteine residues, the first two being separated by said amino acid X.

According to another preferred use said labelled CXC chemokine includes an ELR amino acid sequence therein, wherein E stands for glutamic acid, L stands for leucine and R stands for arginine, and wherein said ELR amino acid sequence immediately precedes a CXC sequence in said chemokine, wherein C stands for cysteine and X stands for an amino acid other than cysteine, said CXC sequence comprising four cysteine residues, the first two being separated by said amino acid X, and wherein said labelled chemokine accumulates at a target site of the animal's body, wherein Interleukin-8 receptor molecules recognizable by said chemokine are located at said target site.

According to still another preferred use the labelled CXC chemokine or labelled peptide accumulates at a target site selected from an inflamed and/or infected portion of said animal's body.

### Brief Description of the Drawings

Fig. 1 is a schematic representation of an Interleukin-8 molecule capable of being utilized in accordance with the present invention.
Fig. 2 is an image of accumulation of indium-111 radiolabelled Interleukin-8 in an inflammatory lesion induced in a thigh of a rat.
Fig. 3 is an image of accumulation of iodine-123 radiolabelled Interleukin-8 in an inflammatory lesion induced in a thigh of a rat.
Fig. 4 is a graph depicting in vitro binding of I-125-IL-8 to human neutrophils.
Fig. 5 is a depiction of gamma camera images of rats injected with inflammation-imaging agents.

### Detailed Description of the Preferred Embodiments

The present invention utilizes Interleukin-8 (IL-8) material, a member of the "CXC" family of chemotactic cytokines (or "chemokines"), an analog, homolog, derivative or fragment thereof, or a peptide having specificity for a receptor of Interleukin-8 (hereinafter sometimes referred to collectively as CXC chemokines, Interleukin-8 material or IL-8 material).

The CXC family of chemotactic cytokines includes, but is not limited to, Interleukin-8, Macrophage Inflammatory Protein-2 (MIP-2), Growth Regulated Gene Products (GRO) including GRO_{α}, GRO_{β} and GRO_{γ}, Melanoma Growth Stimulating Activity (MGSA), Platelet Factor-4 (PF-4), Gamma-interferon Inducible Protein (gamma-IP), Platelet. Basic Protein, Connective Tissue Activating Protein (CTAP-III), Beta-thromoboglobulin (*β*-TG), Neutrophil-activating Peptide-2 (NAP-2), Chicken v-src-inducible Protein (93), and Epithelial Cell-Derived Neutrophil-Activating Factor-78 (ENA-78).

In preferred embodiments, the CXC chemokine utilized is human Interleukin-8, or an analog, homolog, fragment or derivative thereof.

Human IL-8 is approximately 72 amino acid residues in length with a molecular weight of approximately 8 kD, although there are a 77 residue and a few other variant forms.

The cytokine interleukin-8 (IL-8) (also known as neutrophil-activating protein-1 [NAP-1], neutrophil-activating actor [NAF], or neutrophil chemotactic factor [NCF]) is produced by many cell types in response to a variety of inflammatory stimuli, including lipopolysaccharide, interleukin-1, and tumor necrosis factor. It directly mediates neutrophil recruitment, accumulation, and activation. Its effects in vitro resemble those of other neutrophil chemoattractants (C5a, bacterial chemotaxins, leukotrienes, and platelet activating factor). Yet in contrast to these, IL-8 exhibits high specificity for neutrophils; it is stable, and its effects are long-acting but not species-specific in biological systems. IL-8 may represent a common pathway by which other chemotaxins exert their effects in vivo. As noted above, the major form of IL-8 is a polypeptide of 72 amino acid residues, with Mᵣ ~ 8 kDa on SDS-polyacrylamide gel electrophoresis. IL-8 binds with picomolar to nanomolar affinities to abundant receptors (20,- 80,000 per cell) on the plasma membrane of neutrophils, and activates these cells via a GTP-binding protein in a process requiring calcium and protein kinase C. Following binding and activation, IL-8 is internalized and rapidly degraded by the neutrophils.

Members of the "CXC" family of cytokines (CXC chemokines) generally are basic heparin binding polypeptides having proinflammatory and reparative activities. Typical members of the group are 8-10 kD cytokines, and those members other than Interleukin-8 generally having at least about 20-45% homology with Interleukin-8.

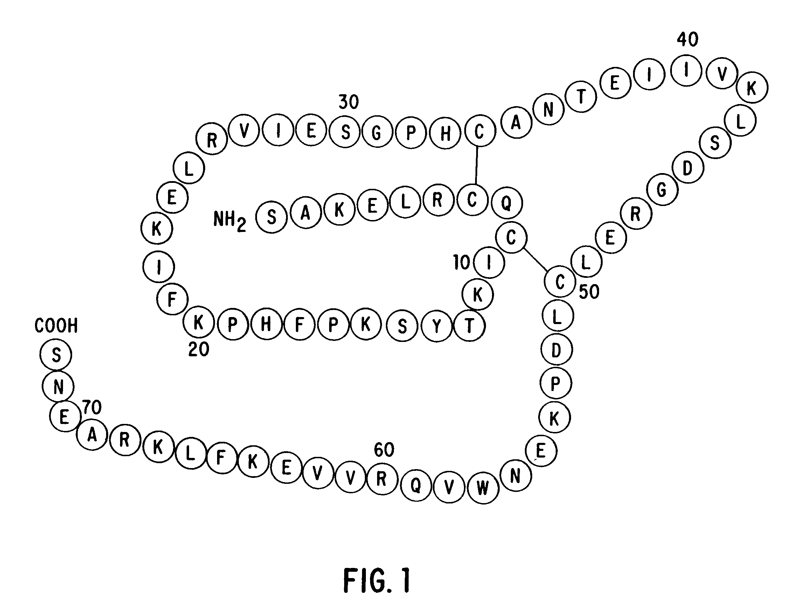

The amino acid sequence of Interleukin-8 is shown in Fig. 1, wherein the circled letters represent amino acids as follows: A represents Alanine, R represents Arginine, N represents Asparagine, D represents Aspartic acid, C represents Cysteine, Q represents Glutamine, E represents Glutamic acid, G represents Glycine, H represents Histidine, I represents Isoleucine, L represents Leucine, K represents Lysine, F represents Phenylalanine, P represents Proline, S represents Serine, T represents Threonine, W represents Tryptophan, Y represents Tyrosine and V represents Valine, or a suitable derivative thereof.

The members of the CXC chemokine family are generally characterized by having an ELR amino acid sequence immediately preceding a conserved C-X-C sequence with four cysteine residues, the first two being separated by a variable amino acid (X) therebetween.

The present invention is further applicable to derivatives of CXC chemokines in which retroinverse or other non-hydrolyzable linkages have been inserted, or D-amino acids substitutions have been made, in order to modify the native L-amino acid-containing sequence.

As noted above, the CXC chemokine carries a label or radioactive agent such as indium, iodine, technetium, rhenium, gallium, samarium, holmium, yttrium, copper, cobalt and the like. In particularly preferred embodiments, the CXC chemokine material carries a radioactive label selected from the group consisting of indium-111, iodine-123 and technetium-99m.

The CXC chemokine can employ any suitable means for carrying the label or radioactive agent. Known methods for labelling peptides include the conventional "post-formed chelate approach" and the more recent "pre-formed chelate approach" developed by Fritzburg et al., U.S. Patent Nos. 4,965,392 and 5,037,630, incorporated herein by reference. In the "pre-formed approach," the chelating agent is complexed with a radionuclide and then conjugated to the peptide. In the "post-formed approach," the chelating agent is first conjugated to the peptide and the resulting conjugate is incubated with radionuclide along with a reducing agent.

Suitable chelating agents for use in the present invention include triamide thiolate (N₃S) chelating agents such as represented by formula (I) and (Ia) below, diamide dithiolate (N₂S₂) chelating agents such as represented by formula (II) below and diamide diphenolic chelating agents such as represented by formula (III) below:

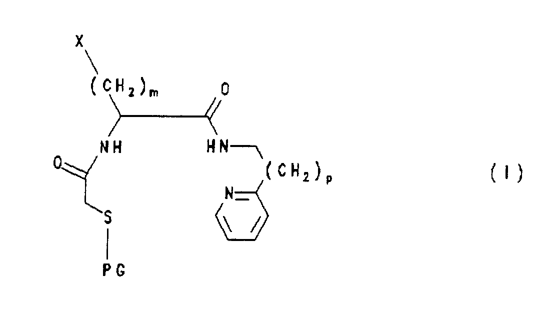

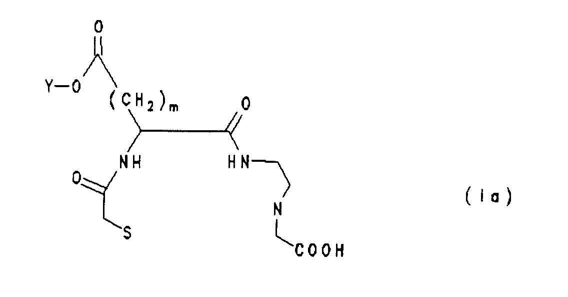

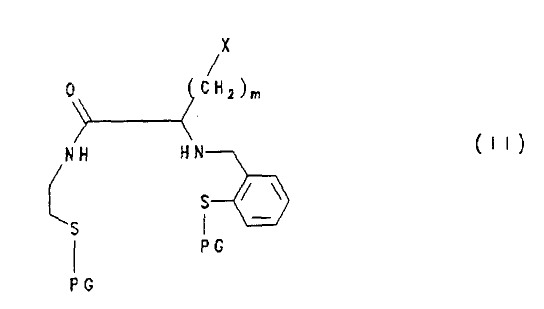

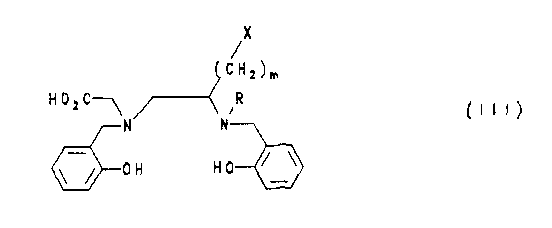

Wherein m in formulas (I), (Ia), (II) and (III) is a whole number of from 1 to about 10 (in formulas (I), (Ia) and (II), m preferably is about 3); P in formula (I) is either 0 or 1; Y of formula (Ia) is o- or p-nitrophenyl, 2-chloro-4-nitrophenyl, cyanomethyl, 2-mercaptopyridyl, hydroxybenztriazole, N-hydroxysuccinimide, trichlorophenyl, tetrafluorophenyl, thiophenyl, tetrafluorothiophenyl, tetrafluorophenyl, thiophenyl, tetrafluorothiophenyl, o-nitro-p-sulfophenyl or N-hydroxyphthalimide, most preferrably tetrafluorophenyl; PG in formulas (I) and (II) is a suitable sulfur protecting group (each of which may be the same or different in formula II) selected from the group consisting of S-acyl groups of from 1 to about 20 carbon atoms such as alkanoyl, benzoyl and substituted benzoyl (wherein alkanoyl is preferred), S-alkyl groups of from 1 to about 20 carbon atoms such as benzyl, t-butyl, trityl, 4-methoxybenzyl and 2,4-dimethoxybenzyl (wherein 2,4-dimethoxybenzyl is preferred), alkoxyalkyl groups of from 1 to about 10 carbon atoms such as methoxymethyl, ethoxyethyl and tetrahydropyranyl (wherein tetrahydropyranyl is preferred), carbamoyl, and alkoxy carbonyl groups of from 1 to about 10 carbon atoms such as t-butoxycarbonyl, methoxycarbonyl and ethoxycarbonyl (wherein t-butoxycarbonyl is preferred); X is a coupling moiety selected from the group consisting of carboxyl, amino, isocyanate, isothiocyanate, imidate, maleimide, chlorocarbonyl, chlorosulfonyl, succinimidyloxycarbonyl, haloacetyl, and N-alkoxycarbamoyl groups of from 1 to about 10 carbon atoms such as N-methyoxycarbamoyl and t-butoxycarbamoyl (wherein in formulas (I) and (II), N-methoxycarbamoyl is preferred); and R of formula (III) is selected from the group consisting of hydrogen and alkyl groups of from 1 to about 10 carbon atoms such as methyl and t-butyl (wherein t-butyl is preferred).

Suitable sulfur-protecting groups, when taken together with the sulfur atom to be protected, include hemithioacetal groups such as ethoxyethyl, tetrahydrofuranyl, methoxymethyl, and tetrahydropyranyl. Other suitable sulfur protecting groups are acyl groups of from 1 to about 20 carbon atoms, preferably alkanoyl or benzoyl. Other possible chelating compounds are described in the European Patent Application assigned publication number 0 284 071.

Synthesis of a radiolabelled chelating agent, such as a Tc-99m bifunctional chelate, and subsequent conjugation to the IL-8 material, can be performed as described in European Patent Application publication number 0 284 071 (supra), U.S. Patent Number 4,965,392 (supra), and related technologies as covered by U.S. patent numbers 4,837,003, 4,732,974 and 4,659,839.

In accordance with one embodiment, the present invention comprises an IL-8 material conjugated with an unlabelled chelating agent, which later can be chelated with a suitable label or radioactive agent.

A lysine of the CXC chemokine can be utilized to carry indium-111. A tyrosine, iodophenyl derivative or lysine (using Bolton-Hunter reagent) of the CXC chemokine can be utilized to carry iodine-123. A lysine or added free cysteine of the CXC chemokine can be utilized to carry technetium-99m.

In another embodiment, the CXC chemokine is labelled with a radioactive agent such as iodine-123 utilizing chloramine-T hydrate.

In still another embodiment, the CXC chemokine is labelled with a radioactive agent such as indium-111 utilizing a chelating agent such as diethylenetriaminepentaacetic acid.

The CXC chemokine carries the label or radioactive agent to a target site of an animal, such as a human or other mammal.

In particularly preferred embodiments, radiolabelled CXC chemokine is injected into an animal's body and allowed to accumulate at a target site of inflammation and/or infection. Interleukin-8 has been found to be a very potent attractant for neutrophils produced by T-lymphocytes, monocytes, vascular endothelium and other tissues. Accordingly, radiolabelled Interleukin-8 is particularly suitable for imaging sites of inflammation and infectious disease in the body. Such sites include Interleukin-8 receptor molecules having areas which are complementary to corresponding Interleukin-8 material.

The radiolabelled CXC chemokine is injected into the subject in a pharmaceutically acceptable carrier, such as an aqueous medium. Generally, a diagnostically effective dosage of radiolabelled CXC chemokine will vary depending on considerations such as age, condition, sex, and extent of disease in the subject individual, counter indications, if any, and variables, to be adjusted by the individual physician. For example, dosage can vary from about 0.01 mg/kg to about 2000 mg/kg, and in more preferred embodiments from about 0.1 mg/kg to about 1000 mg/kg. Initial toxicologic experiments have demonstrated no significant pathology associated with the in vivo administration of IL-8.

The radiolabelled CXC chemokine begins to accumulate within approximately 15 minutes after injection into the subject, and in vivo imaging can be performed utilizing conventional imaging equipment up to 24 hours or more after injection. Known imaging methods include conventional gamma camera techniques, single photon emission computerized tomography (SPECT), and other radionuclide scans.

After accumulating at the target site, the labelled CXC chemokine is gradually cleared from the target site and the animal's system by normal bodily function.

The invention is further illustrated by the following examples, which are not intended to be limiting.

### Example 1

A solution of CXC chemokine such as IL-8 (0.01 mmol), in 2 mL of carbonate/bicarbonate buffer at pH 8.5 ± 0.5 is treated with a solution of 0.1 mmol of the ligand of formula I above (wherein m=2, p=1, PG is benzoyl, and X is succinimidyloxycarbonyl) in dimethylformamide (0.5 mL) and the entire mixture is kept at room temperature for 2 hours. The mixture is then diluted with water (2.5 mL) and dialyzed extensively against water or 0.1 M ammonium bicarbonate, pH 7.5. After dialysis, the solution is lyophilized to give the desired IL-8 conjugate.

### Example 2

A solution of CXC chemokine such as IL-8 (0.01 mmol), in 2 mL of carbonate/bicarbonate buffer at pH 8.5 ± 0.5 is treated with a solution of 0.1 mmol of the ligand of formula (II) above (wherein m=2, both PG are benzoyl, and X is succinimidyloxycarbonyl) in dimethylformamide (0.5 mL) and the entire mixture is kept at room temperature for 2 hours. The mixture is then diluted with water (2.5 mL) and dialyzed extensively against water or 0.1 M ammonium bicarbonate, pH 7.5. After dialysis, the solution is lyophilized to give the desired IL-8 conjugate.

### Example 3

A solution of CXC chemokine such as IL-8 (0.01 mmol) in 2 mL of carbonate/bicarbonate buffer at pH 8.5 ± 0.5 ± 0.5 is treated with a solution of 0.1 mmol of the ligand of formula (III) above (wherein m=4, X is succinimidyloxycarbonyl and R is hydrogen) in dimethylformamide (0.5 mL) and the entire mixture is kept at room temperature for 2 hours. The mixture is then diluted with water (2.5 mL) and dialyzed extensively against water or 0.1 M ammonium bicarbonate, pH 7.5. After dialysis, the solution is lyophilized to give the desired IL-8 conjugate.

### Example 4

To 100 uL of a solution containing 5 mg of sodium gluconate and 0.1 mg of stannous chloride in water, 500 ul of 99m-Tc04 (pertechnetate) is added. After incubation for about 10 minutes at room temperature, a solution of 500 uL of the IL-8 conjugate (1 mg/mL in 0.1 M carbonate/bicarbonate buffer, pH 9.5) of Example 1 or 2 is then added and the entire mixture is incubated at 37°C for about 1 hour. The desired labelled peptide is separated from unreacted 99mTc-gluconate and other small molecular weight impurities by gel filtration chromatography (Sephadex G-50) using phosphate buffered physiological saline, (hereinafter PBS), 0.15M NaCl, pH 7.4 as eluent.

### Example 5

A mixture of gentisic acid (25 mg), inositol (10 mg), and the IL-8 conjugate of Example 3 (500 uL, 1 mg/mL in water) is treated with In-111 indium chloride in 0.05 M HCl. The solution is allowed to incubate at room temperature for about 30 minutes. The desired labelled peptide is separated from unreacted In-111 indium salts and other small molecular weight impurities by gel filtration chromatography (Sephadex G-50) using phosphine buffered physiological saline, (PBS), 0.15M NaCl as eluent.

### Example 6

### Radioiodination of Interleukin-8, chloramine-T hydrate method.

The following reagents and materials are prepared for radioiodination of Interleukin-8:
A) Chloramine-T Hydrate (freshly prepared) (FW = 227.7) (Store stock desiccated, in vacuo, in dark, ambient temperature)
   1. Weigh out 10.0 mg and dilute with 5.0 ml of 0.05 M Phosphate Buffer, pH 6.8.
   2. Take 1.0 ml of dilution and add 9.0 ml 0.05 M Phosphate Buffer, pH 6.8 in volumetric flask.
   3. Concentration = 0.2 mg/ml; use 45 *µ*/exp.=39.5 nmoles.
B) 0.05 M Sodium Phosphate Buffer, Dibasic, pH 6.8. (FW = 268.07) (For Chloramine-T, Metabisulfite, dilution and Reaction).
   1. Weigh out 1.3404 gm and dilute to 80 ml (H₂O).
   2. Adjust pH to 6.8, bring to total volume of 100 ml in volumetric flask.
C) 0.25 M Sodium Phosphate Buffer, Dibasic, pH 6.8. (FW = 268.07) (Reagent for buffering NaI-123 during reaction).
   1. Weigh out 6.7018 gm and dilute to 80 ml (H₂O).
   2. Adjust pH to 6.8, bring to total volume of 100 ml in volmeric flask.
D) Sodium Metabisulfite (Freshly Prepared) (FW = 190.1).
   1. Weigh out 20.0 mg and dilute with 5.0 m of 0.05 M Sodium Phosphate Buffer.
   2. Take 1.0 ml of this solution and add 9.0 ml 0.05 M Sodium Phosphate Buffer.
   3. Concentration = 0.4 mg/ml; use 45 *µ*l=18.0*µ*g/exp.=94.5 nmoles.
E) Phosphate Buffered Saline
   1. Dissolve Sigma (PBS) prepared powder in 1.0 liter of millipore H₂O. Sigma cat. # 1000-3.
   2. Check pH, should be 7.4.
F) BioRad AG1-X8 Anion Resin 100-200 mesh, Acetate form.
   1. Prepare slurry by suspending 6.4 gms of resin in 10 ml PBS.
G) Potassium Iodide Solution (For testing Chloramine-T solution before use in reaction)
   1. Weight out 0.25 gm KI and dilute with 5.0 ml (H₂O).
   2. Add several drops of the Chloramine-T solution to the KI solution.
   3. A color change from a clear to light yellow solution should be observed, if the Chloramine-T solution is reactive.
H) Microfuge Tubes
   1. Sigma siliconized, 1.7 ml, polyproylene, Cat. # T-3406.
I) Sodium Iodine-123
   1. Mallinckrodt Medical, Inc.
J) Interleukin-8 (assuming FW = 8,000)
   1. Use 10 *µ*g per radioiodination = 1.25 nmoles. Pepro Tech Inc.

The following preparations are undertaken prior to running the reaction:

A slurry is prepared of 90% v/v BioRad AG1-X8, 100-200 mesh, acetate form, 24 hours prior to use, with Phosphate Buffered Saline. 2.0 ml of the slurry is poured into a small AG1-X8 column, which then is washed with 10.0 ml Phosphate Buffered Saline. 45.0 *µ*l Chloramine-T (freshly prepared), is pre-drawn into a Hamilton syringe after testing it with 5.0% KI. 45.0 *µ*l Metabisulfite (freshly prepared) is pre-drawn into a Hamilton syringe. 50.0 *µ* l Phosphate Buffered Saline is pre-drawn into a tuberculin syringe, and the syringe is labelled "R". 10.0 ml of Phosphate Buffered Saline is poured into a beaker, and set beside the column, for elution of the column.

Interleukin-8 is labelled with iodine-123 as follows:

To a siliconized microfuge tube reaction vial (1.7 ml), add (12.5 *µ*g) IL-8 in 0.125 ml 0.05 M Phosphate Buffer, pH 6.8. Add (50.0 *µ*l) 0.25 M Phosphate Buffer, pH 6.8 to the reaction vial and gently swirl. Add (20 *µ*l) 20 nanograms "cold" iodide and gently swirl. Add (10-20 *µ*l), 2.0 mCi, NaI-123 to the reaction vial and gently swirl. Add (45.0 *µ*l) of Chloramine-T to the reaction vial, with a pre-drawn Hamilton syringe, and gently swirl. Incubate for 1.5 minutes at room temperature.

Add (45.0 *µ*l) of Metabisulfite to the reaction vial, with a pre-drawn Hamilton syringe, and gently swirl. Assay reaction vial on a Capintec dose calibrator.

After reaction mixture has been assayed, place entire volume on prepared AG1-X8 column. Add (50 *µ*l) PBS to reaction vial and swirl, then add to reaction mixture on column. Unplug column and collect 8 drops in the first tube. Then collect 2 drops in the next 24 tubes. The Vo will be in approximately tube #6. Use 1.7 ml siliconized microfuge tubes. Assay collected fractions on the Capintec dose calibrator. Combine the major fractions starting at the void volume. (NOTE: An aliquot should be taken for a dose assay for accurate results, if tissue distribution studies are being done.) Run TLC of sample in order to observe any free I-123. Spot a very small aliquot on a Gelman ITLC-SG strip, and develop with N-saline for 8 to 10 minutes. Cut into 1.0 cm sections and count on auto-gamma counter. Unreacted 1-123 migrates at or near solvent front, protein remains at origin, small peptides have varying Rf values, at or near the origin.

### Example 7

### Indium-111 Radiolabelling of Interleukin-8

The following reagents and materials are prepared prior to running the reaction:
A) Cyclic-Diethylenetriaminepentaacetic acid dianhydride (C-DTPA), FW - 357.22 is synthesized and kept in a desiccator, in vacuo, at room temperature.
B) Commercially available Dimethylsulfoxide anhydrous (DMSO) is further purified by fractional freezing at or below 18.4°C. 20 to 25 ml of DMSO is placed in an oven dried 100 ml bottle and tightly capped. The bottle is placed in a slurry ice water bath and swirled until liquid solidifies on the walls of the bottle. An oven dried Pasteur pipet is used to remove remaining liquid. The bottle is capped and stored at room temperature under nitrogen, in a desiccator. Aldrich, 27, 685-5.
C) Nitrogen Gass is grade # 5. Airco
D) Phosphate Buffered Saline (PBS)
   1. Dissolve Sigma (PBS) prepared powder in 1.0 liter of millipore water.
   2. Check pH, should be 7.4 Sigma, 1000-3
E) Phosphate Buffered Saline + 5.0% BSA (For equilibration of G-25 column)
   1. To 10.0 ml PBS dissolve 0.5 g. BSA.
   2. Final concentration 50.0 mg/ml BSA.
F) Phosphate Buffered Saline + 0.5% BSA (For elution of G-25 column)
   1. To 50.0 ml PBS dissolve 0125 g BSA.
   2. Final concentration 5.0 mg/ml BSA.
G) G-25, medium grade
   1. Weigh out desired amount and swell in PBS for 24 hours.
   2. Before use degas for 24 or more hours.
   3. 24 hours prior to radiolabelling pour columns and equilibrate and wash with BSA/PBS solutions.
   4. Size of columns to be determined experimentally.
H) 0.10 M diethylenetriaminepentaccetic acid (DTPA), FW = 393.20
   1. Weigh out 0.393 gm DTPA and place in 8.0 ml PBS.
   2. Adjust pH to 5.4, bring to total volume of 10.0 ml in a volumetric flask. (Needs to become acidic for solubility) Sigma, D-6518
I) Millipore water
J) 111-Indium chloride (111-InC13) in 0.05 N HC1 Nordion International, Kanata, Canada, T209A
(NOTE: In order to avoid hydrolysis and trace metal contamination, all reagents should be of the highest purity, DMSO should be purified by fractional freezing, all glassware and instruments should be thoroughly cleaned and rinsed approximately three times with millipore filtered water, and glassware and instruments to be used in the chelation reaction should be oven dried for approximately 24 hours at about 140°C and cooled in a desiccator.)

The reaction is run as follows:

In step 1, C-DTPA (11.075 mg) is dissolved in 5.0 ml anhydrous DMSO. The tube is covered with parafilm, and gently inverted until the solution becomes clear. To 10 *µ*g lyophilized IL-8 is added 0.5 ml PBS, pH 7.4. Add 0.01 ml (22.32 *µ*g) C-DTPA/DMSO from step #1. Incubate solution, at room temperature, for 50 minutes. Gently swirl solution every 15 minutes. After incubation, place entire volume on the prepared G-25 column. Collect 0.20 ml fractions. Combine fractions at Vo and several fractions after the void volume. For Vo determination, use blue dextran. 111-InC13 is assayed on a Capintec dose calibrator and 1.0 to 2.0 mCi of 111-In is pipetted into the IL-8 tube. The tube is gently swirled and assayed for the amount of radioactivity. The 111-In/IL-8 solution is incubated for 30 minutes, at room temperature. Excess 111-In is chelated to prevent the formation of indium hydroxide, an insoluble precipitate. The reaction mixture on a G-25 column and eluted with either PBS or 0.50%- BSA, PBS. 0.20 ml fractions are collected. Fractions are assayed either on the dose calibrator or dilute aliquots of the fractions for counting on the gamma counter. Combine the fractions at the Vo (protein peak) and fractions after the void volume until the radioactivity levels decline. The excess chelated 111-In is eluted at or near the Vt. The combined Vo fractions are assayed on the dose calibrator and the radiolabelling efficiency are calculated. The empty reaction tube is also assayed to assure transfer of the majority of radioactivity.

### Example 8

### Imaging with radiolabelled Interleukin-8

White, male, Sprague-Dawley rats, weighing between 250 and 350 gm were used for imaging of inflammatory lesion. The lesion was induced by injecting 300 *µ*l of 2.0% carrageenan (iota form), suspended in n-saline, into the hind limb. The other hind limb was used as a control and was injected with 300 *µ* l saline. The hind limbs were selected, instead of IP or Sub-Q injections of carrageenan on the body of the rats, because less background activity is present for these studies.

The lesion was induced at various times prior to the injection of the radiolabelled IL-8. A difference in uptake of radioactivity was observed in the carrageenan limb, as compared to the control limb, when the radiolabelled IL-8 was injected 3 hours to 24 hours post lesion induction.

Routinely, images were acquired serially every 15 minutes for 3.0 hours post radiolabel injection. 15 minute images were also acquired every 24 hours post injection, until the labelled material was cleared from the animal.

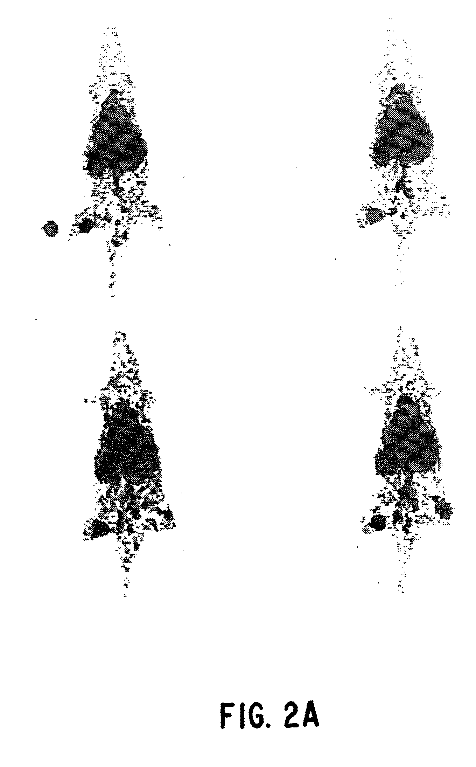

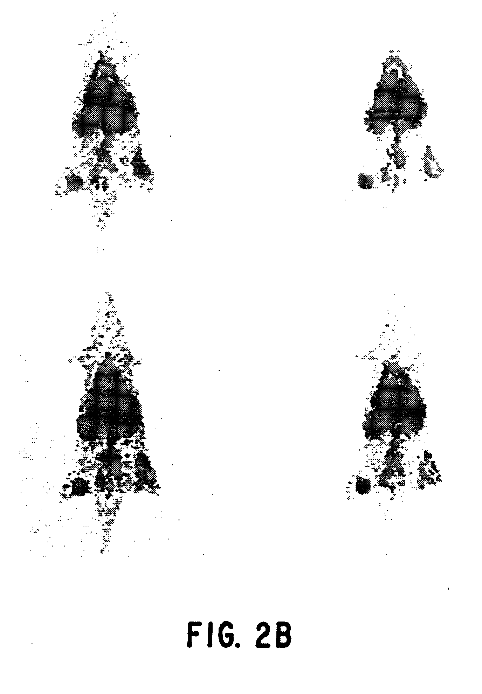

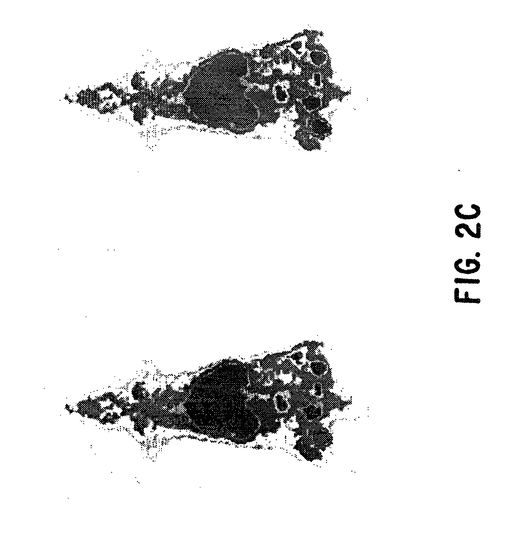

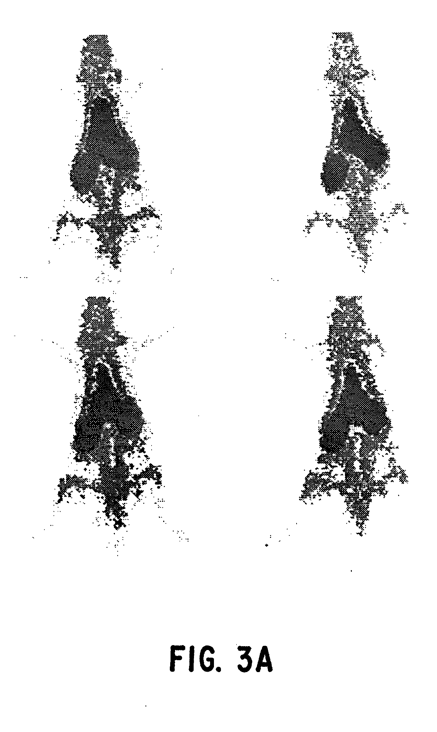

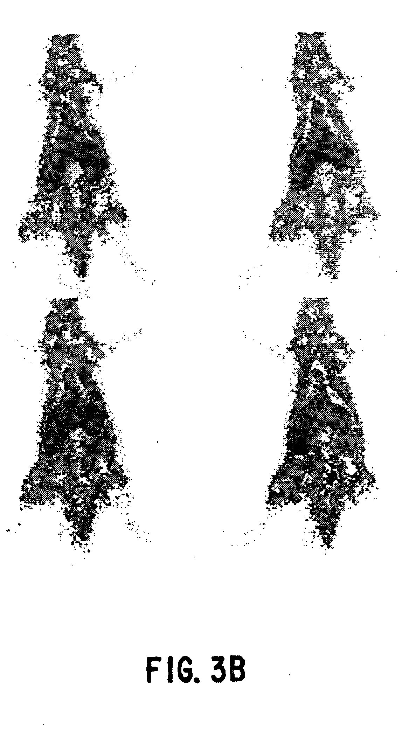

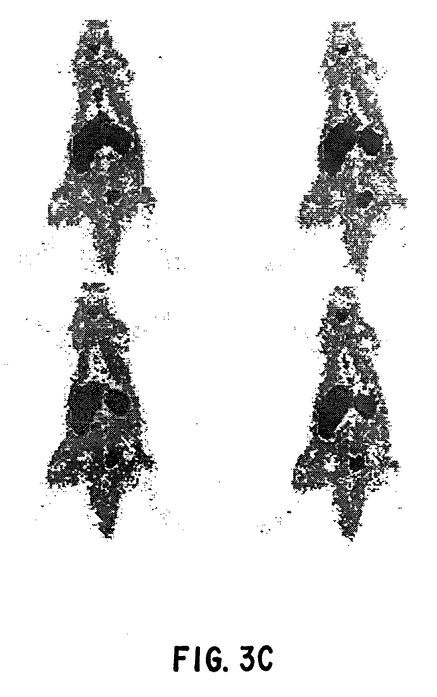

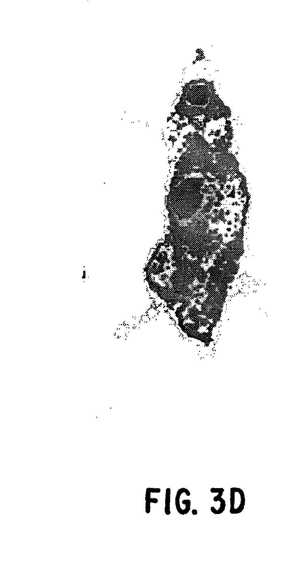

Different cameras and collimators were used, depending on the radionuclide. With 123-I and 111-In, a Siemens, ZLC Orbiter camera and a 140 Kev, high resolution or medium energy collimators were respectively used. Image acquisition and storage, on all cameras, was accomplished by a Siemens MicroDelta computer, connected to a larger MicroVAX unit. Figs. 2 and 3 show accumulation of radiclabelled IL-8 when labeled with In-111 and I-123 respectively.

### Example 9

### Comparison of I-125-labeled human recombinant interleukin-8 (I-125-IL-8) with agents Ga-67 citrate and In-111-labeled human leukocytes for the ability to image a sterile acute inflammatory lesion in the rat hindlimb.

Recombinant human interleukin-8 was obtained from Pepro Tech Inc. I-125 was purchased as NaI from Amersham Corp. Ga-67 citrate and In-111-oxine were obtained from MediPhysics. Tc-99m (obtained from a Mo-99/Tc-99m generator) and TechneScan® PYP® were supplied by Mallinckrodt Medical, Inc. Carrageenan (Type V) was purchased from Sigma Chemical Co. (St. louis, MO). All other reagents and supplies were of the highest grade commercially available.

IL-8 was radioiodinated following recommendations of the radionuclide supplier. Typically, 25-50 *µ*g of IL-8 in 0.1 ml of 0.25 M sodium phosphate, pH 6.8, was added one mass equivalent of non-radioactive NaI in a volume of 0.01 ml, followed by 2 mCi (0.02 ml) of I-125 as sodium iodide and 20 nmol (0.01 ml) of chloramine-T. Reactants were mixed and agitated gently for 90 sec at room temperature. The reaction was quenched by addition of 42 nmol (0.02 ml) of sodium metabisulfite. I-125-IL-8 was separated from nonreacted I-125 by ion exchange on a small column of Bio-Rad AG 1-X8, 50-100 mesh. The recovered product was stored at 4° C until used, and injected within 6 hours after labeling.

Human leukocytes were prepared from a healthy donor and labeled with In-111-oxine, and erythrocytes were labeled in vivo (injecting TechneScan® PYP® and sodium Tc-99m pertechnetate into the tail vein of each rat) following recommendations of the radionuclide suppliers.

Radiolabeling efficiency was determined in a Beckman Gamma 8000 counter, and the proportion of protein-bound 1-125 in the final product was routinely assessed by chromatography on ITLC-SG strips (Gelman) developed in 80% aqueous methanol. SDS-polyacrylamide gel electrophoresis and autoradiography were performed. Chemotaxis of human neutrophils toward nonlabeled IL-8 and I-125-IL-8 was evaluated in a Boyden chamber assay. Specific and nonspecific binding of I-125-IL-8 to isolated human neutrophils were determined.

Male Sprague-Dawley rats (Charles River), 200-250 grams, were housed in small groups under conditions approved by the institutional animal care committee, in compliance with guidelines of the American Association of Laboratory Animal Science. Animals were given ad libitum access to standard rat chow (Purina) and drinking water. Prior to lesion induction, injection of radiopharmaceutical, and imaging, each rat was given up to 13 mg/kg xylazine and 87 mg/kg ketamine subcutaneously in the interscapular region. Additional small doses of ketamine were given as necessary to maintain an adequate level of sedation during the image acquisition.

Acute inflammatory lesions were induced by subcutaneous injection of 4 ml of carrageenan suspension (2% w/v in sterile saline) into the left thigh of each rat. The right (control) thigh was injected with 4 ml of sterile saline. Twenty-four hours later each rat received a single radiopharmaceutical agent, 50-100 *µ* Ci in ≤0.5 ml, by aseptic injection under direct visualization into the right external jugular vein. For the results shown here each inflammation-imaging radiopharmaceutical was injected into four rats, and three rats' erythrocytes were radiolabeled in vivo.

Posterior total body gamma camera images were acquired beginning 15 min following radiopharmaceutical injection and at selected intervals up to 96 hours postinjection. Sedated rats were placed in pairs in their backs atop an inverted camera head with a protective layer over the collimator, and taped to the layer to maintain optimum limb position. Calibration standards corresponding to known fractions of the injected activity for each agent were diluted in scintillation vials to approximate the thickness of the rat thigh, and place upon the covered collimator for concurrent imaging. Images of I-125 and Tc-99m activity were acquired on a Siemens LEM Plus mobile camera with a low energy, high sensitivity collimator, and images of In-11 and Ga-67 activity on a Siemens Orbiter camera using a medium energy collimator. Most acquisitions were obtained over a period of fifteen minutes.

Relative activity was determined by computer-assisted region-of-interest analysis for each hindlimb, for each calibration standard, and for appropriate background regions at each imaging session. Regional counts were converted to percent injected activity corrected for background and for physical decay, and expressed as a function of time postinjecton. Graphical and statistical analysis of the converted data utilized the programs Excel (Microsoft), Minitab (Minitab, Inc.), and Sigmaplot (Jandel Scientific).

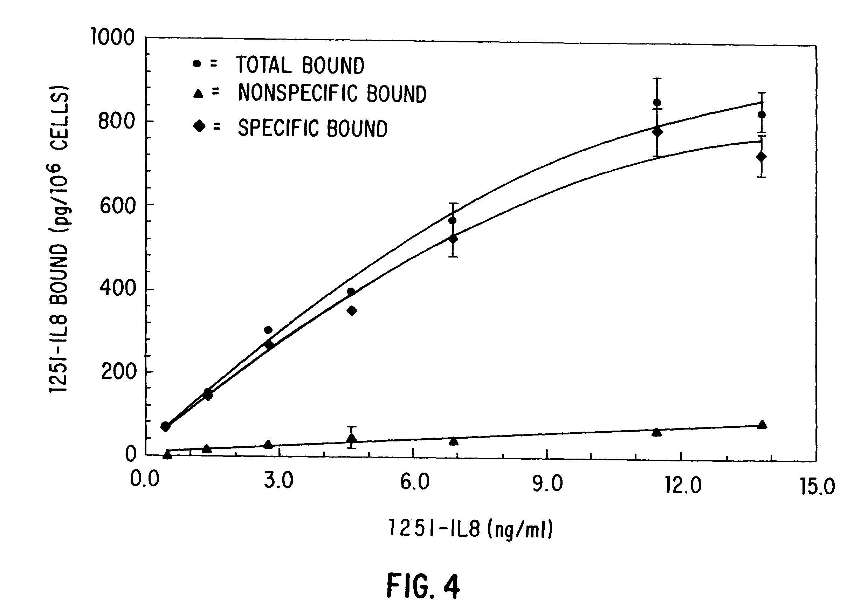

The physical and biological integrity of IL-8 were well preserved during the radiolabeling procedure. Assuming complete physical recovery of IL-8 from the labeling mixture, specific activities of 88-138 CI/mmol were achieved, representing radiolableing efficiencies of 14-45%. Protein-bound radioactivity accounted for 95-98% of the total activity in the final product. The electrophoretic mobility of I-125-IL-8 on SDS-polyacrylamide gels was indistinguishable from that of nonlabled IL-8, and I-125-IL-8 was ≥ 95% as effective as nonlabeled IL-8 with respect to neutrophil chemotaxis when assayed in a Boyden chamber. To asses its receptor-binding properties, we incubated I-125-IL-8 with purified human neutrophils over a 30-fold range of concentrations, both alone (in triplicate) and in the presence of a 1000-fold molar excess of nonlabeled IL-8 (in duplicate) at each concentration (Fig. 4). Specific binding of I-125-IL-8, expressed as the difference between activity bound in the absence and in the presence of competitor, accounted for ≥ 90% of the total binding observed. In agreement with the findings of other investigators, analysis of the binding curves indicated an abundance of high-affinity receptors (estimated maximum of ~60,000 binding sites per cell; k_{d} = 8.3E-10 M).

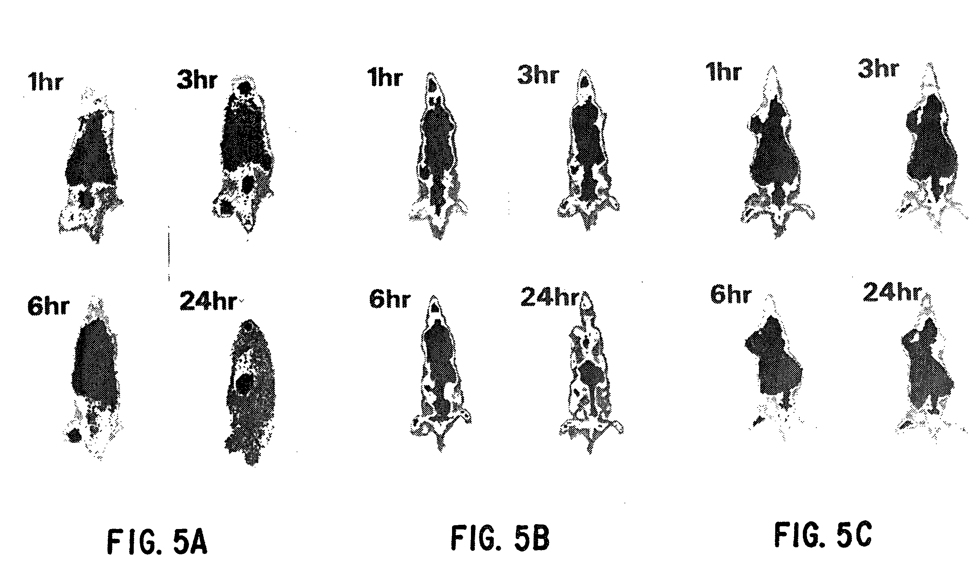

In Figure 5 are displayed posterior images of individual rats obtained 1 to 24 hours following intravenous injection of I-125-IL-8, Ga-67 citrate, and In-111-labeled human leukocytes. These three agents yielded qualitatively similar images, exhibiting asymmetrically increased activity in the left hindlimb compared to the right at all times postinjection.

Persistent asymmetry until at least 96 hours postinjection for all three agents was confirmed. However, the time course of hindlimb activity differed according to the agent given. Left hindlimb I-125-IL-8 activity peaked between one and three hours postinjection, with a calculated means value and standard deviational three hours of 4.8 ± 0.5% of the injected activity corrected for physical decay (%IA), and declined in roughly exponential fashion thereafter. In-111-WBC activity in the left hindlimb also increased rapidly, reaching a value of 4.2 ± 0.6%IA by three hours postinjection and an eventual plateau (4.5 ± 0.4%IA at 24 hours). Left hindlimb Ga-67 activity rose briefly to 9.2 ± 0.5%IA by six hours postinjection, and subsequently declined to a constant level of about 7.5%IA.

For rats injected with I-125-IL-8 or In-111-WBC, left hindlimb activity was significantly greater than right hindlimb activity at all imagining time points (p ≤ 0.05). For the rats injected with Ga-67 citrate, differences between left and right hindlimb activity were significant at all time points except 1 hour postinjection (p + 0.2).

Rats given I-125-IL-8 demonstrated the most marked asymmetry of hindlimb activity partitioning, with a mean left:right hindlimb activity ratio (L/C) of 2.5 ± 0.9 for all imaging time points, and with no significant temporal variation. By analysis of variance, this ratio was significantly higher (p < 0.001) than the corresponding ratio (1.5 ± 0.3) in rats given either Ga-67 citrate or IN-111-WBC.

In order to determine whether differential limb perfusion in response to the inflammatory focus could account for such asymmetry, an additional three carrageenan-treated rats were injected intravenously with stannous pyrophosphate and Tc-99m pertechetate to label their erythrocytes in vivo. Because of the short physical half-life and in vivo degradation of the radiotracer, comparison between this blood pool agent and the inflammation-imaging agents was limited to analysis of images obtained s 24 hours postinjection. As expected, activity in the left hindlimb was highest immediately after erythrocyte labeling (mean 3.1 ± 1.0 %IA), and decline to 1.6 ± 0.5%IA by 24 hours. The mean L/C ration and standard deviation calculated between 0 and 24 hours postinjection for rats carrying Tc-99m-labeled erythrocytes was 1.2 ± 0.2. This ratio is significantly lower than that for rats injected with I-125-IL-8, but surprisingly, it is not significantly different from the L/C ratios of rats receiving Ga-67 citrate or In-111-WBC.

The above demonstrates that I-125-labeled human recombinant interleukin-8 (I-125-IL-8) compares favorably with both Ga-67 citrate and In-111-labeled human leukocytes for imaging of carrageenan-induced acute inflammatory lesions in the rat hindlimb. In this Example, the postinjection time to peak activity for I-125-IL-8 is short, and the target-to-nontarget activity ratio for I-125-IL-8 is significantly higher than that for either agent of comparison or for a radiotracer of blood pool activity.

Without being bound to any particular theory about how I-125-IL-8 selectively appears in the inflammatory focus, the observations suggest that the interactions between IL-8 and neutrophils that have been established through in vitro experimentation may account for the in vivo behavior of I-125-IL-8. Rapid influx of IL-8 into a neutrophil-rich region is entirely consistent with the functioning of a high abundance, high-affinity receptor-mediated process, and the subsequent exponential decline in lesion-associate activity appears logical following rapid internalization and degradation of the IL-8 ligand.

Initially, there was consideration of radiolabeling homologous rat leukocytes for comparison in this Example, but our attempts to prepare In-111-labeled leukocytes from normal rat blood resulted in unsatisfactory cell yield and labeling efficiency. Wishing neither to confound our experimental model by infusing exogenously stimulated leukocytes nor to exsanguinate rats needlessly, we elected to use readily available normal human leukocytes.

IL-8 offers several a priori advantages for clinical nuclear imagining. As a naturally occurring human polypeptide commercially available in recombinant (and potentially synthetic form), it is both contagion-free and nonimmunogenic, without known toxic effects in humans (unlike bacterial cytokines). IL-8 is readily radioiodinated without detectably altering its physical or biological properties, and preliminary work shows that IL-8 may also be radiolabeled with I-123 or In-111 to yield qualitatively comparable images in carrageenan-treated rats to those presented here. Radiolabeling and administration of IL-8 involve no handling of blood or time-consuming isolation of blood components, considerations that would make IL-8 an agent of choice in individuals harboring blood-borne transmissible infections, and in subjects for whom an urgent study is indicated. The size and solubility of IL-8 permit ready access to tissue neutrophils, with a minimal lag period expected between injection and satisfactory imageability. Finally, the affinity and high receptor number on neutrophils for IL-8 should result in considerable signal amplification in an acute inflammatory focus of infectious or noninfectious etiology. This feature would make radiolabeled IL-8 particularly useful for detecting focal accumulation of neutrophils in neutropenic subjects and small children, from whom comparatively large blood volumes must be drawn to isolate peripheral leukocytes for a direct radiolabeling procedure.

Interleukin-8 material, when carrying a label or radioactive agent in accordance with the present invention, is particularly suitable for imaging sites of inflammation and infectious disease. Since many modifications, variations and changes in detail may be made to the described embodiments, it is intended that all matter in the foregoing description and shown in the accompanying drawings be interpreted as illustrative and not in a limiting sense.

## Claims

1. The use of a labelled CXC chemokine or labelled peptide having specificity for a receptor of Interleukin-8 for preparing a pharmaceutical composition for imaging a target site in an animal body, said target site including Interleukin-8 receptor molecules.

2. The use of claim 1 wherein said target site is an inflamed or diseased portion of said animal's body.

3. The use of claim 1 wherein said labelled CXC chemokine is radiolabelled CXC chemokine.

4. The use of claim 3 wherein said CXC chemokine is labelled with indium-111, iodine-123, technetium-99m or copper-62.

5. The use of claim 4 wherein said indium-111 is carried by a lysine of said CXC chemokine.

6. The use of claim 4 wherein said CXC chemokine is labelled with indium-111 utilizing a chelating agent.

7. The use of claim 6 wherein said chelating agent is C-DTPA.

8. The use of claim 6 wherein said CXC chemokine is human Interleukin-8.

9. The use of claim 4 wherein said iodine-123 is carried by a tyrosine, iodophenyl derivative or lysine of said CXC chemokine.

10. The use of claim 4 wherein said CXC chemokine is labelled with iodine-123 utilizing chloramine-T hydrate.

11. The use of claim 10 wherein said CXC chemokine is human Interleukin-8.

12. The use of claim 4 wherein said CXC chemokine is labelled with technetium-99m.

13. The use of claim 12 wherein said technetium-99m is carried by a lysine or an added free cysteine of said CXC chemokine.

14. The use of claim 1 wherein said CXC chemokine is labelled utilizing a chelating agent.

15. The use of claim 14 wherein said chelating agent is a triamide thiolate ligand, a diamide dithiolate ligand or a diamide diphenolic ligand.

16. The use of claim 15 wherein said CXC chemokine is labelled with technetium-99m or copper-62 and said chelating agent is a triamide thiolate ligand.

17. The use of claim 12 wherein said CXC chemokine is human Interleukin-8.

18. The use of claim 1 wherein said CXC chemokine is human Interleukin-8.

19. The use of a CXC chemokine or peptide carrying a labeling agent, which peptide recognizes Interleukin-8 receptor molecules of said CXC chemokine, for preparing a pharmaceutical composition for delivering a labeling agent to a target site of an animal's body by introducing into an animal's body said CXC chemokine or peptide carrying a labeling agent, and allowing the labeling agent-carrying CXC chemokine or peptide to accumulate at a target site which includes Interleukin-8 receptor molecules so as to deliver said labeling agent to said target site.

20. The use of claim 19, wherein said labeling agent is a radioactive agent.

21. The use of anyone of claims 1, 19 or 20 wherein said CXC chemokine is Neutrophil-activating Peptide-2 (NAP-2).

22. A composition comprising a CXC chemokine carrying a labeling agent selected from the group consisting of indium-111, iodine-123 and technetium-99m.

23. A composition comprising CXC chemokine conjugated with a chelating agent.

24. The composition of claim 23 wherein said chelating agent is a triamide thiolate ligand, diamide dithiolate ligand or diamide diphenolic ligand.

25. The use of claim 1, wherein said chemokine is a CXC chemokine including an ELR amino acid sequence, wherein E stands for glutamic acid, L stands for leucine and R stands for arginine, and wherein said ELR amino acid sequence immediately precedes a CXC sequence in said chemokine, wherein C stands for cysteine and X stands for an amino acid other than cysteine, said CXC sequence comprising four cysteine residues, the first two being separated by said amino acid X.

26. The use of claim 1, wherein the labelled CXC chemokine includes an ELR amino acid sequence therein, wherein E stands for glutamic acid, L stands for leucine and R stands for arginine, and wherein said ELR amino acid sequence immediately precedes a CXC sequence in said chemokine, wherein C stands for cysteine and X stands for an amino acid other than cysteine, said CXC sequence comprising four cysteine residues, the first two being separated by said amino acid X, and wherein said labelled chemokine accumulates at a target site of the animal's body, wherein Interleukin-8 receptor molecules recognizable by said chemokine are located at said target site.

27. The use of claim 1, wherein the labelled CXC chemokine or labelled peptide accumulates at a target site selected from the inflamed and/or infected portion of said animal's body.

28. The use of claim 27, wherein labelling is performed as defined in anyone of claims 3 to 7, 9, 10 and 12 to 16.

## Patentansprüche

1. Verwendung eines markierten CXC Chemokins oder eines markierten Peptids mit Spezifität für einen Rezeptor von Interleukin-8 zur Herstellung einer pharmazeutischen Zusammensetzung zur Abbildung eines Target-Bereichs im Körper eines Lebewesens, wobei der Target-Bereich Interleukin-8-Rezeptormoleküle umfasst.

2. Verwendung nach Anspruch 1, worin der Targetbereich ein entzündeter oder erkrankter Abschnitt des Körpers des Lebewesens ist.

3. Verwendung nach Anspruch 1, worin das markierte CXC Chemokin ein radiomarkiertes CXC Chemokin ist.

4. Verwendung nach Anspruch 3, worin das CXC Chemokin mit Indium-111, Jod-123, Technetium-99m oder Kupfer-62 markiert ist.

5. Verwendung nach Anspruch 4, worin Indium-111 durch ein Lysin des CXC Chemokins getragen wird.

6. Verwendung nach Anspruch 4, worin das CXC Chemokin unter Verwendung eines chelatbildenden Mittels mit Indium-111 markiert ist.

7. Verwendung nach Anspruch 6, worin das chelatbildende Mittel C-DTPA ist.

8. Verwendung nach Anspruch 6, worin das CXC Chemokin humanes Interleukin-8 ist.

9. Verwendung nach Anspruch 4, worin das Jod-123 von einem Tyrosin, einem Jodophenylderivat oder einem Lysin des CXC Chemokins getragen wird.

10. Verwendung nach Anspruch 4, worin das CXC Chemokin mit Jod-123 unter Verwendung von Chloramin-T-Hydrat markiert ist.

11. Verwendung nach Anspruch 10, worin das CXC Chemokin humanes Interleukin-8 ist.

12. Verwendung nach Anspruch 4, worin das CXC Chemokin mit Technetium-99m markiert ist.

13. Verwendung nach Anspruch 12, worin das Technetium-99m von einem Lysin oder einem addierten freien Cystein des CXC Chemokins getragen wird.

14. Verwendung nach Anspruch 1, worin das CXC Chemokin unter Verwendung eines chelatbildenden Mittels markiert ist.

15. Verwendung nach Anspruch 14, worin das chelatbildende Mittel ein Triamidthiolatligand, ein Diamiddithiolatligand oder ein Diamiddiphenolligand ist.

16. Verwendung nach Anspruch 15, worin das CXC Chemokin mit Technetium-99m oder Kupfer-62 markiert ist und das chelatbildende Mittel ein Triamidthiolatligand ist.

17. Verwendung nach Anspruch 12, worin das CXC Chemokin humanes Interleukin-8 ist.

18. Verwendung nach Anspruch 1, worin das CXC Chemokin humanes Interleukin-8 ist.

19. Verwendung eines ein Markierungmittel tragenden CXC Chemokins oder Peptids, wobei das Peptid Interleukin-8-Rezeptormoleküle des CXC Chemokins erkennt, zur Herstellung einer pharmazeutischen Zusammensetzung zur Übertragung eines Markierungsmittels auf den Target-Bereich des Körpers eines Lebewesens, wobei man in den Körper des Lebewesens das das Markierungsmittel tragende CXC Chemokin oder das Peptid einführt und man das das Markierungsmittel tragende CXC Chemokin oder Peptid im Target-Bereich, welcher Interleukin-8-Rezeptormoleküle enthält, akkumulieren lässt, um so das Markierungsmittel zum Target-Bereich zu bringen.

20. Verwendung nach Anspruch 19, wobei das Markierungsmittel ein radioaktives Agens ist.

21. Verwendung nach einem der Ansprüche 1, 19 oder 20, worin das CXC Chemokin das Neutrophil-aktivierende Peptid-2 (NAP-2) ist.

22. Zusammensetzung, umfassend ein CXC Chemokin, welches ein Markierungsmittel trägt, das ausgewählt ist unter Indium-111, Jod-123 und Technetium-99m.

23. Zusammensetzung, umfassend ein CXC Chemokin, konjugiert mit einem chelatbildenden Mittel.

24. Zusammensetzung nach Anspruch 23, worin das chelatbildende Mittel ein Triamidthiolatligand, ein Diamiddithiolatligand oder ein Diamiddiphenolligand ist.

25. Verwendung nach Anspruch 1, worin das Chemokin ein CXC Chemokin ist, welches eine Aminosäuresequenz ELR umfasst, worin E für Glutaminsäure, L für Leucin und R für Arginin steht, und wobei die Aminosäuresequenz ELR der CXC-Sequenz in dem Chemokin, worin C für Cystein und X für eine andere Aminosäure als Cystein steht, unmittelbar vorausgeht, wobei die CXC-Sequenz 4 Cysteinreste umfasst, wobei die ersten beiden durch die Aminosäure X getrennt sind.

26. Verwendung nach Anspruch 1, worin das markierte CXC Chemokin eine Aminosäuresequenz ELR umfasst, worin E für Glutaminsäure, L für Leucin und R für Arginin steht, und wobei die Aminosäuresequenz ELR der CXC-Sequenz in dem Chemokin, worin C für Cystein und X für eine andere Aminosäure als Cystein steht, unmittelbar vorausgeht, wobei die CXC-Sequenz 4 Cysteinreste umfasst, wobei die ersten beiden durch die Aminosäure X getrennt sind, und worin das markierte Chemokin im Targetbereich des Körpers des Lebewesens akkumuliert, wobei Interleukin-8-Rezeptormoleküle, welche durch das Chemokin erkennbar sind, im Target-Bereich lokalisiert sind.

27. Verwendung nach Anspruch 1, worin das markierte Chemokin oder markierte Polypeptid in einem Target-Bereich akkumuliert werden, welcher ausgewählt ist unter entzündeten und/oder infizierten Abschnitten des Körpers des Lebewesens.

28. Verwendung nach Anspruch 27, worin die Markierung gemäß der Definition in einem der Ansprüche 3 bis 7, 9, 10 und 12 bis 16 durchgeführt wird.

## Revendications

1. Utilisation d'une chemokine CXC marquée ou d'un peptide marqué ayant une spécificité pour un récepteur d'interleukine-8 en vue de préparer une composition pharmaceutique destinée à visualiser un site cible dans le corps d'un animal, ledit site cible incluant des molécules de récepteur d'interleukine-8.

2. Utilisation selon la revendication 1, **caractérisée en ce que** ledit site cible est une partie enflammée ou malade dudit corps de l'animal.

3. Utilisation selon la revendication 1, **caractérisée en ce que** ladite chemokine CXC marquée est une chemokine CXC marquée par un radio-isotope.

4. Utilisation selon la revendication 3, **caractérisée en ce que** ladite chemokine CXC est marquée par l'indium-111, l'iode-123, le technétium-99m ou le cuivre-62.

5. Utilisation selon la revendication 4, **caractérisée en ce que** ledit indium-111 est porté par une lysine de ladite chemokine CXC.

6. Utilisation selon la revendication 4, **caractérisée en ce que** ladite chemokine CXC est marquée par l'indium-111 en utilisant un agent chelatant.

7. Utilisation selon la revendication 6, **caractérisée en ce que** ledit agent chelatant est c-DTPA.

8. Utilisation selon la revendication 6, **caractérisée en ce que** ladite chemokine CXC est une interleukine-8 humaine.

9. Utilisation selon la revendication 4, **caractérisée en ce que** ledit iode-123 est porté par une tyrosine, un dérivé iodophényle ou une lysine de ladite chemokine CXC.

10. Utilisation selon la revendication 4, **caractérisée en ce que** ladite chemokine CXC est marquée avec l'iode-123 en utilisant un hydrate de chloramine-T.

11. Utilisation selon la revendication 10, **caractérisée en ce que** ladite chemokine CXC est une interleukine-8 humaine.

12. Utilisation selon la revendication 4, **caractérisée en ce que** la chemokine CXC est marquée par le technétium-99m.

13. Utilisation selon la revendication 12, **caractérisée en ce que** ledit technétium-99m est porté par une lysine ou une cystéine libre ajoutée de ladite chemokine CXC.

14. Utilisation selon la revendication 1, **caractérisée en ce que** ladite chemokine CXC est marquée en utilisant un agent chelatant.

15. Utilisation selon la revendication 14, **caractérisée en ce que** ledit agent chelatant est un ligand triamide thiolate, un ligand diamide dithiolate ou un ligand diamide diphénolique.

16. Utilisation selon la revendication 15, **caractérisée en ce que** ladite chemokine CXC est marquée par le technétium-99m ou le cuivre-62 et ledit agent chelatant est un ligand triamide thiolate.

17. Utilisation selon la revendication 12, **caractérisée en ce que** ladite chemokine CXC est une interleukine-8 humaine.

18. Utilisation selon la revendication 1, **caractérisée en ce que** ladite chemokine CXC est une interleukine-8 humaine.

19. Utilisation d'une chemokine CXC ou d'un peptide portant un agent marquant, lequel peptide reconnaît les molécules de récepteur d'interleukine-8 de ladite chemokine CXC en vue de préparer une composition pharmaceutique destinée à délivrer un agent marquant à un site cible du corps d'un animal en introduisant dans le corps d'un animal ladite chemokine CXC ou ledit peptide portant un agent marquant, et en permettant à la chemokine CXC ou au peptide portant l'agent marquant de s'accumuler au niveau du site cible qui inclut des molécules de récepteur d'interleukine-8 de façon à délivrer ledit agent marquant audit site cible.

20. Utilisation selon la revendication 19, **caractérisée en ce que** l'agent marquant est un agent radioactif.

21. Utilisation selon l'une quelconque des revendications 1, 19 ou 20, **caractérisée en ce que** ladite chemokine CXC est un peptide-2 activant les neutrophiles (NAP-2).

22. Composition comprenant une chemokine CXC portant un agent marquant sélectionné dans le groupe constitué par l'indium-111, l'iode-123 et le technétium-99m.

23. Composition comprenant la chemokine CXC conjuguée à un agent chelatant.

24. Composition selon la revendication 23, **caractérisée en ce que** ledit agent chelatant est un ligand triamide thiolate, un ligand diamide dithiolate ou un ligand diamide diphénolique.

25. Utilisation selon la revendication 1, **caractérisée en ce que** ladite chemokine est une chemokine CXC incluant une. séquence aminoacide ELR, dans laquelle E représente l'acide glutamique, L représente la leucine et R représente l'arginine, et en ce que ladite séquence aminoacide ELR précède immédiatement une séquence CXC dans ladite chemokine, dans laquelle C représente la cystèine et X représente un acide aminé autre que la cystéine, ladite séquence CXC comprenant quatre résidus cystéine, les deux premiers étant séparés par ledit acide aminé X.

26. Utilisation selon la revendication 1, **caractérisée en ce que** la chemokine CXC marquée inclut une séquence aminoacide ELR, dans laquelle E représente l'acide glutamique, L représente la leucine et R représente l'arginine, en ce que ladite séquence aminoacide ELR précède immédiatement la séquence CXC dans ladite chemokine, dans laquelle C représente la cystéine et X représente un acide aminé autre que la cystéine, ladite séquence CXC comprenant quatre résidus cystéine, les deux premiers étant séparés par ledit acide aminé X, et en ce que ladite chemokine marquée s'accumule au niveau d'un site cible du corps de l'animal, dans lequel les molécules de récepteur d'interleukine-8 reconnaissable par ladite chemokine sont situées au niveau du site cible.

27. Utilisation selon la revendication 1, **caractérisée en ce que** ladite chemokine CXC marquée ou ledit peptide marqué s'accumule au niveau d'un site cible choisi pour être étant une partie enflammée et/ou infectée dudit corps de l'animal

28. Utilisation selon la revendication 27, **caractérisée en ce que** le marquage est effectué comme défini dans l'une quelconque des revendications 3 à 7, 9, 10 et 12 à 16.
